# EUROPEAN PATENT APPLICATION

(11) **EP 2 048 534 A1**
(43) Date of publication of application: **15.04.2009**
(21) Application number: 07803652.2
(22) Date of filing: 27.07.2007
(51) Int. Cl.: G02B 21/34, C03C 25/66, G01N 33/48, G01N 1/28

(54) **METHOD FOR THE SURFACE TREATMENT OF SOLID SUBSTRATES**

(30) Priority: 28.07.2006 ES 200602057
(71) Applicant: Universidad del Pais Vasco, 48940 Leioa Vizcaya (ES); FUNDACION TEKNIKER, 20600 Eibar-Guipuzcoa (ES)
(72) Inventor: RODRÍGUEZ PUERTAS, Rafael, E-48940 Leioa - Vizcaya (ES); BAREDA GÓMEZ, Gabriel, E-48940 Leioa - Vizcaya (ES); FERNÁNDEZ GONZÁLEZ, José Andrés, E-48940 Leioa - Vizcaya (ES); ASTIGARRAGA ARRIBAS, Egoitz, E-48940 Leioa - Vizcaya (ES); ARANZABE GARCÍA, Ana, E-20600 Eibar - Guipúzcoa (ES); MARCAIDE RODRÍGUEZ, Arrate, E-20600 Eibar - Guipúzcoa (ES); GÓMEZ PLAZA, David, E-20600 Eibar - Guipúzcoa (ES)
(74) Representative: ABG Patentes, S.L.
(86) International application number: PCT/ES2007/000465
(87) International publication number: WO 2008/012391

(57) **Abstract**

The invention relates to a process for the chemical modification of a surface of a support, in which said surface contains available hydroxyl groups and lacks metals or metallic alloys. According to the invention, the surface is treated with orthophosphoric acid. The surface thus treated can be used to immobilize biomolecules, cellular membranes, liposomes, cells or tissue sections. The invention is suitable for use in biotechnology, proteomics, genomics, pharmacology and histology in order to carry out studies on immobilized products.

## Description

### Field of the Invention

The present invention is encompassed within the technology of immobilization of products (biomolecules, cell membranes, liposomes, cells or tissues) on solid supports, and, specifically, in methods based on the chemical modification of the surfaces of said solid supports for immobilizing said products.

The invention is applicable, among others, in the areas of biotechnology, proteomics, genomics, pharmacology and histology, since it allows performing any type of studies on the immobilized products.

### Background of the Invention

The immobilization of biomolecules is a process by which the degrees of freedom of movement of biomolecules are completely or partially restricted by means of their binding to a support insoluble in water. The main aspects to be considered in the immobilization of biomolecules are the selected support and mechanism of immobilization.

The choice of the support is a determining factor since it must be sought that the immobilization increases affinity for the substrate, reduces inhibition, extends the optimal pH range and reduces the possibilities of microbial contamination. Furthermore, the support must have mechanical resistance suitable to the operating conditions of the reactor and must be easily separable from the liquid medium so that it can be reused.

The supports used until now are organic (natural polymers and synthetic polymers) or inorganic types. Organic type supports have the drawback of offering little thermal stability. The most used inorganic type supports are the supports manufactured with metal oxides (gold, titanium, silicon, etc.) and glass [Kikukawa T. et al., (2005), Journal of Membrane Science, vol. 259, 161-166; Shin D. et al., (2003), Biosensors and Bioelectronics, 19, 485-494].

Regarding immobilization methods, adsorption is one of the simplest methods. By means of this method, the biomolecule is bound to a non-functionalized support by means s of ionic interactions, Van der Waals forces and/or hydrogen bridges. In these types of methods, several compounds such as polylysine and gelatin are used. These methods have the advantage of their low cost, simple preparation and stability of the derivatives in work media with low water content. Nevertheless, their drawbacks include the little stability of the derivatives from the mechanical point of view, a weak binding to the support and the use of measures for conserving the coated supports.

The most used methods of immobilization by chemical binding are those which occur by means of the formation of covalent binding and are based on the use of coupling agents which are covalently bound to the support by on one hand and, on the other, to the biomolecule to be immobilized through functional groups. Several coupling agents are known for carrying out the covalent binding to the support and immobilizing the biomolecule depending on the available reactive functional groups, amino, thiol, aldehyde, epoxy groups, etc.

The functional amino group is the first option since it is the most general chemical coupling agent. Most macromolecules contain amino groups which can be used as chemical coupling agents. These types of coupling agents are less suitable in the case of acid ligands in which the amino group forms an active center and in the case of molecules having many amino groups.

The possibility of using thiol as a functional coupling group primarily depends on the availability of the thiol groups in the ligand. The chemical binding of thiol is stronger than that of amine; therefore, the conditions of the functional group are less critical. The coupling agents based on thiol functional groups cannot be used under strong reducing conditions, since the disulfide bond is instable in said conditions.

The aldehyde functional group is used in specific cases, for example, with polysaccharides and glycoconjugates having cis-cis-diols and with salicylic acid. These groups are quite readily oxidized by aldehydes.

The epoxy functional group allows a suitable reaction with different thermoplastic organic matrices and considerable bond stability over time.

Silane-derivatives are a widely used group of coupling agents. In this type of derivatives, one end of the molecule (the group silane) is covalently bound to the inert support and the other end containing the chemical functional group, e.g., the amino, thiol, aldehyde, epoxy group, etc., is bound to the biomolecule to be immobilized.

In other cases, when the previous options are inappropriate, the streptavidin-biotin binding pair is used.

These types of methods generally have some advantages, such as the stability of the immobilized biomolecules and a high level of control of the immobilization of the biomolecules, and some drawbacks since the processes are laborious, costly, highly corrosive compounds are used and their use depends on the nature of the biomolecule to be immobilized.

Although different methods have been described for the treatment of surfaces of supports intended for the immobilization of biomolecules, there is still a great need for designing alternative methods that allow providing supports with the desired optical and mechanical properties, in a simple and economically suitable manner.

### Summary of the Invention

The present invention provides an alternative process for the treatment of solid supports, such as supports intended for the immobilization of different types of products (e.g., biomolecules, cell membranes, liposomes, cells or tissue sections), comprising the treatment of said supports with orthophosphoric acid. The surfaces of the supports that can be subjected to said treatment have available hydroxyl groups and lack metals or metallic alloys. Illustrative examples of supports that can be subjected to said treatment include supports the surfaces of which are of glass or of a polymeric material, etc., provided they meet said conditions of the presence of available hydroxyl groups and absence of metals or metallic alloys.

The process for the treatment of surfaces provided by this invention is an alternative to silanization, is simple, allows obtaining homogeneous surfaces, does not have conservation problems and uses compounds which are more acceptable from the environmental point of view. It further allows its application on supports, e.g., microscope slides, of standard size and composition which are used for multiple applications in the fields of biomedicine and biotechnology.

Therefore, an object of the present invention is a process for the chemical modification of the surface of a support comprising the treatment of said surface with orthophosphoric acid. Said support can be a suitable solid support for immobilizing different types of products (e.g., biomolecules, cell membranes, liposomes, cells, tissue sections, etc.).

The surface of said support chemically modified according to the previously described process, as well as the supports which incorporate it, are additional aspects of the present invention.

In another aspect, the invention relates to a solid support, such as a solid support for immobilizing said different types of products, comprising a surface treated according to the previously described process. In a particular embodiment, said surface is a glass surface, such as the surface of a glass microscope slide.

In another aspect, the invention relates to a method for immobilizing a product (e.g., biomolecules, cell membranes, liposomes, cells, tissue sections, etc.) in a solid support comprising a surface treated according to the previously described process of chemical modification of surfaces, said method comprising the step of placing said product in contact with said surface treated according to the previously described process.

In another aspect, the invention relates to a solid support comprising a surface treated according to the previously described process of chemical modification of surfaces and at least one product (e.g., b i omolecules, cell membranes, liposomes, cells, tissue sections, etc.) immobilized on the treated surface of said solid support.

### Brief Description of the Drawings

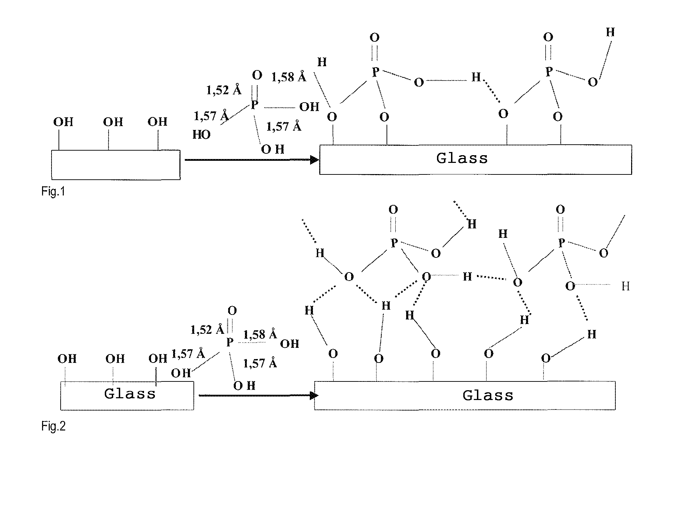

Figure 1 is a schematic representation illustrating the type of covalent binding of the orthophosphoric acid to a surface of a support containing available hydroxyl groups, e.g., glass.
Figure 2 is a schematic representation showing the type of binding by means of hydrogen bridges of the orthophosphoric acid to a surface of a support containing available hydroxyl groups, e.g., glass.

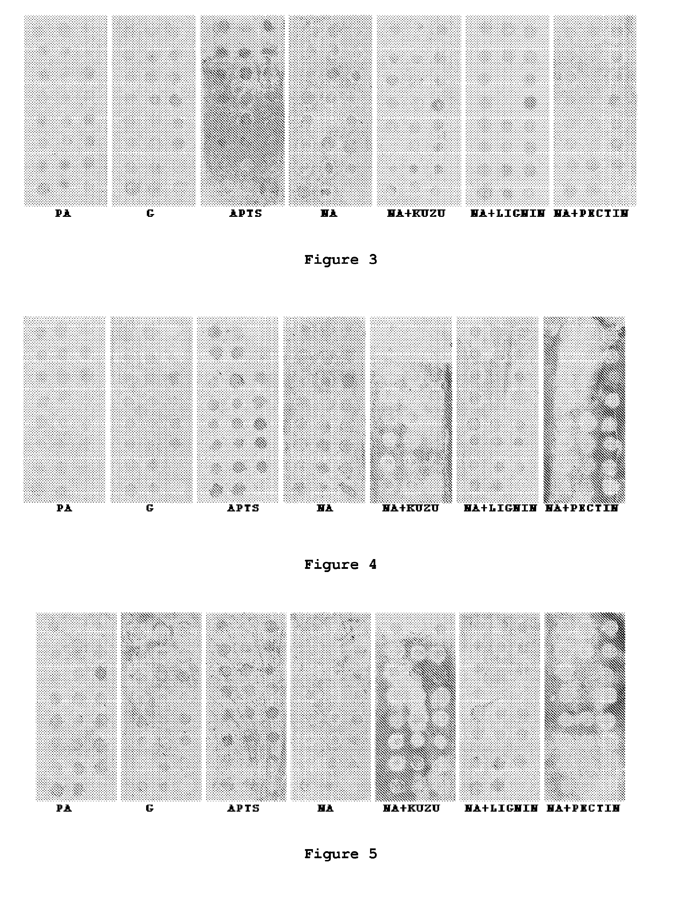

Figure 3 illustrates the immobilization of isolated cell membranes and shows the stimulation of the basal binding of [³⁵S]GTPgammaS ([³⁵S]GTPγS) in tissue arrays in the absence of a ligand. The results obtained using glass microscope slides subjected to different treatments, specifically to treatment with phosphoric acid (PA), gelatin (G), gamma-aminopropyltriethoxysilane (APTS), nitric acid and gelatin (NA), nitric acid and Kuzu (NA+KUZU), nitric acid and lignin (NA+LIGNIN) or nitric acid and pectin (NA+PECTIN), are shown.
Figure 4 illustrates the immobilization of isolated cell membranes and shows the stimulation of the binding of [³⁵S]GTPγS in tissue arrays in the presence of the cannabinoid agonist WIN-55,212-2 [Example 2]. The results obtained by subjecting glass microscope slides to different treatments, specifically to treatment with phosphoric acid (PA), gelatin (G), APTS, nitric acid and gelatin (NA), nitric acid and Kuzu (NA+KUZU), nitric acid and lignin (NA+LIGNIN) or nitric acid and pectin (NA+PECTIN), are shown.
Figure 5 illustrates the immobilization of isolated cell membranes and shows the stimulation of the binding of [³⁵S]GTPγS in tissue arrays in the presence of the muscarinic agonist carbachol. The results obtained by subjecting glass microscope slides to different treatments, specifically to treatment with phosphoric acid (PA), gelatin (G), APTS, nitric acid and gelatin (NA), nitric acid and Kuzu (NA+KUZU), nitric acid and lignin (NA+LIGNIN) or nitric acid and pectin (NA+PECTIN), are shown.

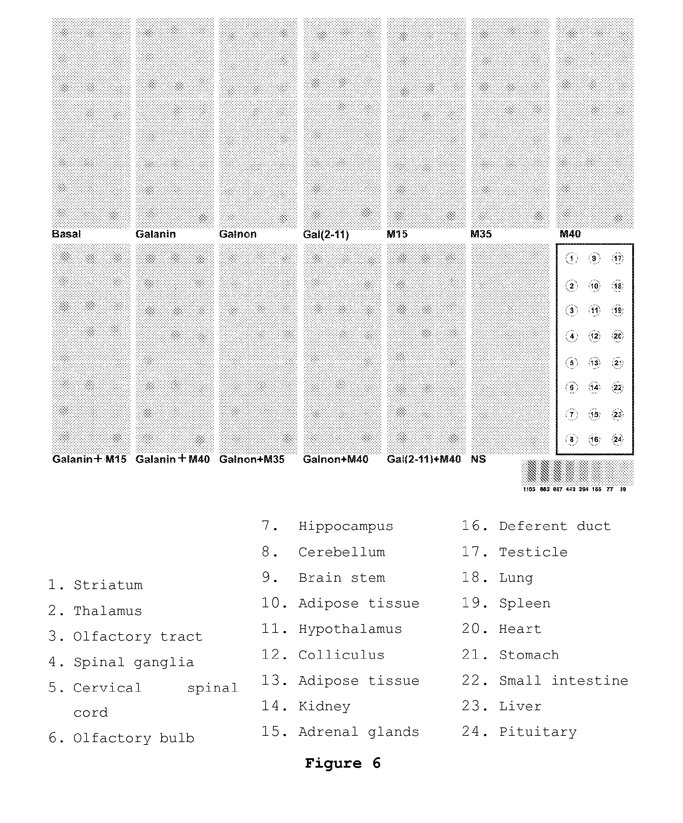

Figure 6 illustrates the immobilization of isolated cell membranes and shows the binding of [³⁵S]GTPγS in the absence (basal) and in the presence of different ligands of galanin [Galanin, Galnon, Gal(2-11), M15, M35, M40 and combinations thereof, such as Galanin+M15, Galanin+M40, Galnon+M35, Galnon+M40 and Gal(2-11)+M40] receptors. The non-specific binding is determined in the presence of 10 µM GTPγS (NS). The ¹⁴C standard and the values in nCi/gt.e assigned to each color are shown in the lower right corner.

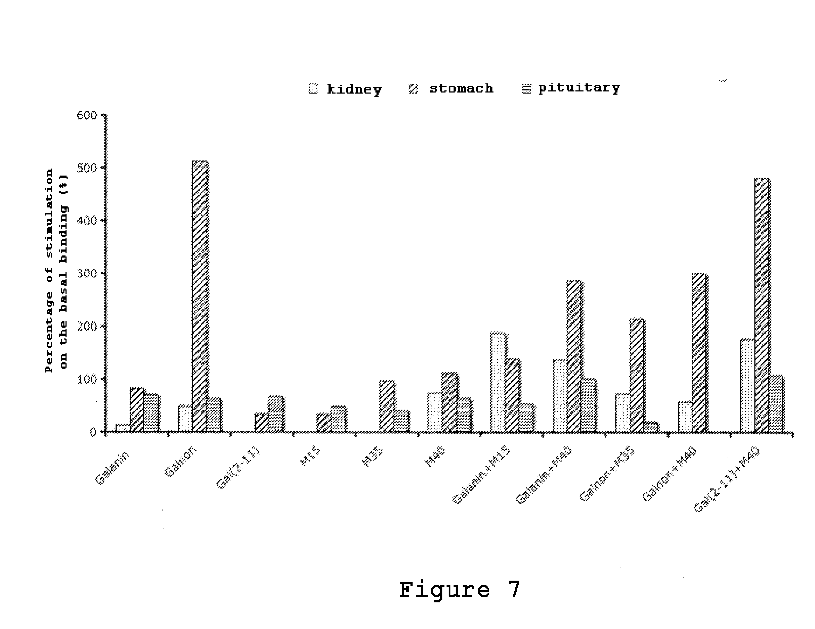

Figure 7 is a representative bar diagram of the percentages of stimulation on the basal binding of [³⁵S]GTPγS in the presence of different ligands of galanin [Galanin, Galnon, Gal(2-11), M15, M35, M40 and combinations thereof, such as Galanin+M15, Galanin+M40, Galnon+M35, Galnon+M40 and Gal(2-11)+M40] receptors in the kidney, stomach and pituitary quantified in tissue arrays.

### Detailed Description of the Invention

In one aspect, the invention is aimed at a process for the chemical modification of the surface of a support, hereinafter "process of the invention", comprising subjecting said surface to treatment with orthophosphoric acid.

The supports that can be subjected to said treatment are supports the surfaces of which have the following characteristics:
(i) they contain available hydroxyl groups (i.e., they have -OH groups capable of reacting with orthophosphoric acid); and
(ii)they lack metals or metallic alloys (due to the fact that the orthophosphoric acid attacks metals and metallic alloys).

Said supports can be used for immobilizing different types of products, for example, biomolecules, cell membranes, liposomes, cells, tissue sections, etc.

Non-limiting, illustrative examples of supports that can be subjected to the treatment with orthophosphoric acid according to the present invention include supports the surfaces of which are totally or partially formed by glass or by a polymeric material which meets the previously mentioned conditions (i) and (ii), e.g., plastic material, (poly)methacrylate, etc. The ample offer existing on the market and its low cost make glass an interesting support material for the immobilization of biomolecules.

Said support can have any shape and size suitable for the purposes for which they are intended; nevertheless, in a particular embodiment, said support has a smooth or substantially planar and uniform dimensional surface. In a particular embodiment, said support is a microscope slide, of standard size and composition, of the type used in biomedicine and biotechnology applications.

Therefore, in a particular embodiment, said support comprises a surface formed by glass. Virtually any borosilicate-type glass surface commonly used as laboratory material can be modified by means of the treatment with orthophosphoric acid proposed by this invention to provide it with affinity and capacity for immobilizing different types of products, for example, biomolecules, cell membranes, liposomes, cells, tissue sections, etc. Said glass surface can form part of a solid support or of a device or apparatus, forming all of it or a part of the same. In a particular embodiment, said glass surface is in the form of a glass microscope slide (support) of the type commonly used in biomedical or biotechnological applications.

Orthophosphoric acid (PO₄H₃) is a known and commercially available product having a tetrahedral structure. During the treatment of the surface of the support with orthophosphoric acid two competitive reactions occur, a covalent binding through the hydroxyl groups present in the surface of the support with elimination of water on one hand, and an adherence to said surface containing hydroxyl groups by the formation of hydrogen bridges (Figures 1 and 2) on the other. As a result of both reactions, which include the formation of covalent bonds, binding by hydrogen bridges and elimination of water, homogeneous surfaces coated with orthophosphoric acid with strong hydrogen bridges are achieved. Thus, a reduction of the hydrophilia of the surface of the support occurs, providing it with high affinity and capacity of immobilizing different types of products, for example, biomolecules, cell membranes, liposomes, cells, tissue sections, etc.

The concentration of the orthophosphoric acid used for putting the process of the invention into practice can vary within a wide range. In a particular embodiment, orthophosphoric acid in aqueous solution with a concentration comprised between approximately 10% by weight and approximately 85% by weight, preferably between 40% by weight and 85% by weight, is used. In a specific embodiment, concentrated orthophosphoric acid (85% by weight) in aqueous solution is used. For putting the process of the invention into practice, the surface of the support to be treated is placed in contact with orthophosphoric acid. The treatment of said surface with orthophosphoric acid is carried out at a temperature comprised between approximately 20°C and approximately 100°C, preferably between 20°C and 60°C, for a time period comprised between 2 and 24 hours, preferably between 5 and 15 hours.

If desired, the process of the invention further comprises one or more steps prior to the treatment with phosphoric acid for the purpose of washing and/or degreasing the surface to be treated.

Therefore, in a particular embodiment, before the treatment with orthophosphoric acid, the surface of the support to be treated is placed in contact with a solvent, such as an organic solvent, to wash and/or degrease said surface, and is then dried. To that end, said solvent is applied on the surface of the support to be treated by any conventional method, for example, by means of spraying, sprinkling, etc., or the surface of the support is immersed in said organic solvent. Any inert solvent could be used as a solvent for the surface to be treated, i.e., which does not attack or adversely affect said surface; advantageously, said solvent has a great degreasing power and a high evaporation rate, such as a short-chain alcohol or ketone, for example, an alcohol or a ketone of 1-6 carbon atoms, preferably, an alcohol or a ketone of 1-4 carbon atoms, e.g., ethanol, isopropanol, acetone, etc. In a specific embodiment, the surface to be treated is a glass surface and the solvent is selected from a ketone (e.g., acetone) and an alcohol (e.g., isopropanol). The subsequent drying of the surface or of the support can be performed by conventional methods, for example, by means of a synthetic airflow or in an oven. In a particular embodiment, the drying is performed by means of a synthetic airflow at a pressure comprised between approximately 0.1 bar (10⁴ Pa) and approximately 0.01 bar (10⁵ Pa), preferably between 0.3 bar (3x10⁴ Pa) and 0.6 bar (6x10⁴ Pa), for a time period comprised between approximately 1 minute and approximately 10 minutes, preferably between 2 and 6 minutes. The synthetic air must not contain more than 3 ppm of water or more than 3 ppm of total hydrocarbons [THC] (such as CH₄). The drying by means of an oven is carried out at a temperature comprised between approximately 100°C and approximately 160°C, preferably between 110°C and 140°C, for a time period comprised between approximately 30 minutes and approximately 3 hours, preferably between 1 and 2 hours. In a specific embodiment, the organic solvent used in this treatment is acetone.

In another particular embodiment, after said first washing of the surface of the support with an organic solvent and drying, a second washing of said surface is carried out, for which purpose the surface of the support to be treated is placed in contact with a second organic solvent and is then dried. To that end, the second organic solvent is applied on the surface of the support to be treated by any conventional method, for example, by means of spraying, sprinkling, etc., or said surface is immersed in said organic solvent. As previously mentioned, said solvent must not attack the surface of the support to be treated and, advantageously, it must have a great degreasing power and a high evaporation rate; non-limiting, illustrative examples of solvents which meet such conditions include short-chain alcohols and ketones, for example, alcohols or ketones of 1-6 carbon atoms, preferably, of 1-4 carbon atoms, e.g., ethanol, isopropanol, acetone, etc. The subsequent drying of the surface or of the support can be performed by conventional methods, for example, by means of a synthetic airflow or in an oven. In a particular embodiment, the drying is performed by means of a synthetic airflow at a pressure comprised between approximately 0.1 bar (10⁴ Pa) and approximately 0.01 bar (10⁵ Pa), preferably between 0.3 bar (3x10⁴ Pa) and 0.6 bar (6x10⁴ Pa), for a time period comprised between approximately 1 minute and approximately 10 minutes, preferably between 2 and 6 minutes. The synthetic air must not contain more than 3 ppm of water or more than 3 ppm of THC (as CH₄). The drying by means of an oven is carried out at a temperature comprised between approximately 100°C and approximately 160°C, preferably between 110°C and 140°C, for a time period comprised between approximately 30 minutes and approximately 3 hours, preferably between 1 and 2 hours. In a specific embodiment, the organic solvent used in this second washing is isopropanol, advantageously, anhydrous isopropanol.

The solid support obtained according to the process of the invention, comprising a surface treated according to the process of the invention, is an additional aspect of the invention. In a particular embodiment, said solid support comprising a surface treated according to the process of the invention is a glass microscope slide.

By means of the process of the invention, chemically modifying the surface of the support thus treated is achieved, providing it with affinity and capacity for immobilizing different types of products, for example, biomolecules, cell membranes, liposomes, cells, tissue sections, etc.

Therefore, in another aspect, the invention relates to a method for immobilizing a product in a surface of a solid support, in which said product is selected from a biomolecule, a cell membrane, a liposome, a cell, a tissue section, and mixtures thereof, and said surface contains available hydroxyl groups and lacks metals or metallic alloys, said method comprising the step of placing said product in contact with said surface treated according to the process of the invention.

The nature of the product to be immobilized on the surface of the solid support can be quite varied, for example, a biomolecule, a cell membrane, a liposome, a cell or a tissue section. If desired and possible, different products can be combined in one and the same support.

The term "biomolecule", as it is herein used, relates to an organic or inorganic molecule, both those forming and those generated by living beings, and includes polynucleotides or nucleic acids (e.g., single-strand or double-strand DNA, RNA, etc.), proteins (e.g., enzymes, hormones, immunoglobulins, antibodies, ribozymes, etc.), lipids (e.g., fatty acids, triglycerides, phospholipids, glycolipids, etc.), combinations of lipids with proteins and/or with polynucleotides (e.g., cholesterol associated to low density lipoproteins (LDL-cholesterol), cholesterol associated to high density lipoproteins (HDL-cholesterol), etc), substrates or cofactors of the previously mentioned compounds (e.g., sugars, coenzymes, metabolites, etc.), etc.

Alternatively, the product to be immobilized on the surface treated according to the process of the invention includes cell membranes (e.g., fragments of plasma membranes or of membranes of cell organelles), liposomes (of any type), cells (e.g., wild type, transformed, transfected, etc.), tissue sections (fragments), etc.

In a particular embodiment, said biomolecule is a polynucleotide, a protein, a lipid, a combination of a lipid with a protein and/or with a polynucleotide, a substrate or cofactor of the previously mentioned compounds, a fragment of a cell membrane, a liposome, a cell or a tissue section, susceptible of being immobilized for performing assays which involve the existence and/or detection of specific chemical reactions [e.g., binding of antibodies or radioligands to membrane molecules, studies of activity of receptors and of their effector systems: activation of G proteins, adenylyl cyclase, phospholipases, etc., as well as the determination of second messengers such as 3'-5'-cyclic adenosine monophosphate (cAMP), inositol 3 phosphate (IP3), diacylglycerol (DAG), 3'-5'-cyclic guanosine monophosphate (cGMP), etc.].

For putting said method for immobilizing different types of products (e.g., biomolecules, cell membranes, liposomes, cells or tissue sections) on a treated surface of a solid support into practice, the product to be immobilized is placed in contact with said surface treated according to the process of the invention (hereinafter, "treated surface" or "surface treated") under conditions which allow the immobilization of said product on said treated surface by means of conventional techniques, for example, by means of spraying, sprinkling, etc. of a solution or suspension of said product on the treated surface and left to act for a time period, at a suitable temperature (depending on the product to be immobilized, on the solvent it is in if in solution or suspension, etc); alternatively, said treated surface (or the solid support which contains it) is immersed in a solution or suspension of the product to be immobilized for a suitable time period so that said product is immobilized on the treated surface. After the application of the product on the treated surface, the combination (immobilized product)-(treated surface), is left to dry for the purpose of consolidating the binding of the product to the treated surface contained in said solid support (time and temperature will vary depending on the type of product which is intended to be immobilized and on the studies in which said samples will be used).

In a particular embodiment, the product to be immobilized is a biomolecule, e.g., a polynucleotide, a protein, a lipid, a combination of a lipid with a protein and/or with a polynucleotide, or a substrate or cofactor of the previously mentioned compounds. Said biomolecule can either be in the form of solution or suspension in a suitable medium (e.g., in solid state or lyophilized, forming part of liposomes or cell membranes, dissolved or suspended in assay buffer, distilled water, organic solvents, etc.).

In another particular embodiment, the product to be immobilized is a biomolecule, cell membrane (e.g., a cell membrane (plasma or of a cell organelle) from a cell tissue, a membrane of a transfected cell, a membrane of an optionally transformed microorganism, a lipid membrane, etc.), a liposome, a cell or a tissue section. In a specific embodiment, said product to be immobilized is in solid state or in the form of a suspension in a suitable medium (e.g., in solid state or lyophilized, forming part of liposomes or cell membranes, dissolved or suspended in assay buffer, distilled water, organic solvents, etc.).

The method for immobilizing products provided by this invention allows immobilizing products by means of their binding to a surface previously treated with orthophosphoric acid contained in a solid support strongly enough, but without affecting the active centers of said product. Said method for immobilizing different types of products provided by this invention is a simple, low cost method and does not require the application of special measures for conserving the treated solid supports.

Likewise, in another aspect, the invention relates to a solid support comprising a surface treated according to the process of the invention and a product immobilized on said treated surface, wherein said product is selected from a biomolecule, a cell membrane, a liposome, a cell, a tissue section and mixtures thereof. Said solid support comprising said product immobilized on said treated surface can be obtained by means of a method for immobilizing products such as that previously described. In a particular embodiment, said solid support comprising a product immobilized on said treated surface is a glass microscope slide, e.g., a glass microscope slide of the type commonly used in microscopy.

The following examples are useful for illustrating the invention and, for this reason, must not be considered to limit the scope of the same.

### EXAMPLE 1

### Treatment of a glass surface

Glass microscope slides (MENZEL®) with the characteristics that are mentioned below were used as a support and concentrated orthophosphoric acid was used as a chemical modifier.

### Characteristics of the glass microscope slides

Chemical composition:
SiO₂: 72.20%
Na₂O: 14.30%
K₂O: 1.20%
CaO: 6.40%
MgO: 4.30%
Al₂O₃: 1.20%
Fe₂O₃: 0.03%
SO₃: 0.30%
Mean coefficient of expansion: 90.3 x 10⁻⁷/°C (20-300°C)
Refraction rate: 1.513-1.523 (measured in the range of 546.7 nm-643.85 nm) Density: 2.47+0.01 kg/dm³

### Characteristics of the chemical modifier

Orthophosphoric acid was used as a chemical modifier. The characteristics of said chemical modifier were the following:
PANREAC® 85% orthophosphoric acid
Maximum limit of impurities:
Content: 85-88%
Precipitable substances by NH₄OH: not specified
Hypophosphorous and phosphorous acid: not specified
Chlorides: 0.005%
Nitrates: not specified
Sulfates: 0.01%
Heavy metals (in Pb): 0.001%
As: 0.0002%
Cu: 0.001%
Mg: 0.01%
Pb: 0.001%
Ca: 0.01%
Fe: 0.005%
Ni: 0.001%

### Treatment of the glass microscope slides with the chemical modifier

The glass microscope slides were subjected to the following treatment:
1. Immersion of the glass microscope slides (in vertical position) in acetone (chemical grade) for 5-10 minutes.
2. Drying of the glass microscope slides with air.
3. Immersion of the glass microscope slides (in vertical position) in anhydrous isopropanol for 5-10 minutes.
4. Drying of the glass microscope slides with air.
5. Immersion of the glass microscope slides (in vertical position) in concentrated orthophosphoric acid (85%) for 24 hours.
6. Washing with distilled water (Millipore).
7. Air drying.

### Verification of the functionalization of the treated glass surface

The contact angle (θ) with water was measured to verify the functionalization of the surfaces of the glass microscope slides. The contact angle (θ) is defined as the angle formed between the surface and the line tangent to the point of contact between the drop of liquid and the surface of the substrate. The value of said angle depends, among others, on the surface energy of the substrate (the adhered layer of orthophosphoric acid) and on the surface tension of the test liquid (in this case, the test liquid used was deionized water or DI water).

The measurement of the contact angle provides information on the degree of hydrophilia/hydrophobia that a surface has when placed in contact with a liquid. It is generally said that a liquid wets (it is hydrophilic) a solid when the contact angle is less than 90° whereas, in the opposite case, a liquid does not wet (it is hydrophobic) when the contact angle is greater than 90°. Nevertheless, it is very common to refer to "degrees of hydrophilia or of wetting", i.e., a surface A is more hydrophobic/hydrophilic than a surface B if the contact angle (θ) of said Surface A is higher/lower than that of surface B".

The variation of the contact angle (θ) through the chemical modification of surfaces means that the surface energy of the surface has changed, i.e., the tendency that the solid surface [glass microscope slides (in this case)] has of attracting or repelling molecules of other substance (e.g., biomolecules) has changed. For example, when the contact angle (θ) increases compared with that of the reference surface [untreated MENZEL® glass microscope slides], a reduction of the surface energy of the solid surface was achieved; the surface has less force or capacity of attracting other molecules of the test liquid, and therefore the liquid wets less, and, accordingly, the contact angle (θ) is higher.

SURFTENS universal equipment was used to measure the contact angle (θ). The equipment consists of an optical bench, translators operated by micrometric screws, a video camera and a light source. To that end, briefly, the contact angle (θ) was determined by placing a small drop of DI water on the surface of the glass microscope slides with a syringe. The drop was illumined with a diffuse light source to produce an image of clear edges and the produced image was captured with a video camera. Then the contact angle (θ) formed by the liquid on the solid surface was automatically measured using specialized software.

The obtained results were the following:
- the contact angle (θ) formed by the DI water on the glass surface treated with orthophosphoric acid according to the previously described process was 30.2°, whereas
- the contact angle (θ) formed by the DI water on the untreated glass surface (reference surface) was 16.1°.

These results clearly show that the chemical modification of the surface of the glass microscope slides by means of treatment with orthophosphoric acid has caused a variation in the surface energy of the substrate (the adhered layer of orthophosphoric acid) which means that the surface of the glass microscope slides treated with orthophosphoric acid is more hydrophobic (30.2°) than the untreated surface of the glass microscope slides (16.1°).

### EXAMPLE 2

### Immobilization of isolated cell membranes

The efficacy of the method of treating solid supports intended for the immobilization of biomolecules provided by this invention has been verified by means of the immobilization of isolated cell membranes.

### Materials and Methods

### Animals

Five male Sprague Dawley rats (250-275 g) were anesthetized and sacrificed according to the guide approved by the Ethics Committee of the Faculty of Medicine of the Basque Country University following internationally accepted guidelines (86/609/EEC). Organs and tissues which would be used in the experiment (pancreas, stomach, small intestine, spleen, heart, lung, liver, testicle, deferent duct, kidney, suprarenal glands, spinal cord and brain: frontal cortex, striatum, brain stem, pituitary, olfactory nuclei, hippocampus, hypothalamus, thalamus, colliculus and cerebellum) were subsequently extracted. These samples were stored at -70°C until performing the experiment.

### Preparation of membranes

The tissue samples (1 g) were homogenized using a Teflon-glass homogenizer (10 taps at 1,500 rpm) in 30 volumes of homogenization buffer 1 mM [ethylene glycol-bis(β-aminoethyl)-N,N,N',N'-tetraacetic acid (EGTA), 3 mM MgCl₂, 1 m M dithiothreitol (DTT) and 50 mM Tris-HC1, pH 7.4] supplemented with 0.25 mM sucrose. The crude homogenate was centrifuged at 3,000 x g and at 4°C for 5 minutes and the resulting supernatant was again centrifuged at 40,000 x g for 10 minutes (4°C). The pellet was washed in 20 volumes of homogenization buffer and recentrifuged in the same conditions. Aliquots were prepared and stored at -70°C. The protein content was determined according to the Bradford method [Bradford MM., Anal Biochem 1976; 72:248-54] using bovine serum albumin (BSA) as standard.

### Preparation of the tissue arrays

Aliquots of 1 mg/ml of each tissue were taken and with this suspension, 1 µl microspots were made on glass supports (microscope slides) subjected to the following treatments:
a) Treated with orthophosphoric acid (PA) according to the process of Example 1;
b) Gelatinized (G);
c) Coated with gamma-aminopropyltriethoxysilane (APTS);
d) Treated with nitric acid and gelatinized (NA);
e) Treated with nitric acid and subsequently with lignin (NA+lignin);
f) Treated with nitric acid and subsequently with pectin (NA+pectin); or
g) Treated with nitric acid and subsequently with Kuzu (NA+Kuzu).

The lignin (Lignin, Organosolv, Ref. 8068-03-9, Aldrich Chemical Company Inc.), pectin (Apple Pectin Powder, Ref. 0120. Solgar Laboratoires, USA) and Kuzu (Mitoku Organic Kuzu) used in the treatment of the glass microscope slides are commercial products.

The arrays were frozen at -70°C until performing the experiment.

### Autoradiography of [³⁵S]GTPγS

The microscope slides with the tissue sections were left drying for 15 minutes at room temperature. Subsequently the tissue sections were preincubated in assay buffer at pH 7.4 [50 mM Tris-HCl, 3 mM MgCl₂, 0.2 mM EGTA, 100 mM NaCl, 3 mU/ml adenosine deaminase] for 20 minutes at room temperature to eliminate, as much as possible, the presence of endogenous neurotransmitters; after that, the sections were again preincubated in the assay buffer supplemented with guanosine diphosphate (GDP) (2 mM) and DTT (1 mM).

The microscope slides were then incubated for 2 hours at 30°C in the previous assay buffer, to which the following substances were added depending on the different conditions of the experiment:
Basal: [³⁵S]GTPγS 0.04 nM
Activated: [³⁵S]GTPγS 0.04 nM + agonist
Inhibited: [³⁵S]GTPγS 0.04 nM + agonist + antagonist
Nonspecific: [³⁵S]GTPγS 0.04 nM + GTPγS 10 µM

After this time, 2 15-minute washings were performed in 50 mM Tris-HCl buffer, pH 7.4 at 4°C. After the second washing, the sections were immersed in distilled water at 4°C to eliminate the salts and the microscope slides were dried by means of the application of a cold airflow.

Subsequently, the microscope slides with the corresponding sections were exposed to a radiosensitive film (Kodak Biomax MR) which was developed similarly to photographic film. The labeling was quantified transforming the different gray densities into nCi/g tissue equivalent. To that end, each film was exposed together with previously calibrated commercial standards (Amersham). The quantification was performed with microscopic anatomical resolution in a computerized image analysis system, using NIH-image software.

The substances added in the different assays were the following:
(A) To study the binding of [³⁵S]GTPγS in the absence (basal) and in the presence of agonists, the following agonists were used:
   (i) the cannabinoid agonist WIN 55212-2 [(R)-(+)-[2,3-dihydro-5-methyl-3-[(4-morpholinyl)methyl]pyrrolo[1,2,3-d,e]-1.4-benzoxazin-6-yl]-(1-naphthalenyl) methanone monomethanesulphonate] [D'Ambra TE et al. (1992) J Med Chem. 135(1):124-35; Kuster JE, et al., (1993) J Pharmacol Exp Ther. 264(3):1352-63], product supplied by Tocris Cookson (Bristol, United Kingdom); and
   (ii) the muscarinic agonist carbachol (H₂N-C(O)O-(CH₂)₂-N(CH₃)₃)⁺ (Cl)⁻ (Merck Index 13th Edition Merck and Co., Inc.; Reference No. 1786); and
(B)To study the binding of [³⁵S]GTPγS in the absence (basal) and in the presence of ligands of galanin receptors, the following ligands were used: galanin, galnon, gal(2-11), M15 [galanin-(1-13)-substance P-5-11 amide], M35 [galanin-(1-13)-bradykinin,-(2,9) amide] and M40 [galanin-(1-13)-Pro-Pro-Ala-Leu-Ala-Leu-Ala-Leu-Ala amide].

### Results

### Binding of [³⁵S]GTPγS in the absence (basal) and in the presence of agonists

Figure 3 shows the results of the basal binding of [³⁵S]GTPγS; in said Figure, it can be observed how the supports treated with orthophosphoric acid or with nitric acid and subsequent treatment with lignin or Kuzu have a lower nonspecific binding than with other supports (e.g., APTS). Nevertheless, when the same experiment is performed in the presence of the cannabinoid agonist WIN 55212-2 (Figure 4), it is observed that the microscope slides with lower nonspecific binding are those treated with orthophosphoric acid. This effect is also observed in the experiment performed in the presence of the muscarinic agonist carbachol (Figure 5). Furthermore, it can be observed that the deposits of membranes on this support have fewer halos than the remaining supports used, and that the percentage of effect in the microscope slides treated with orthophosphoric acid [treatment a)] is maximum. Another observed advantage was that the adherence of the sample was maintained during the entire experimental process in the microscope slides treated with orthophosphoric acid. This same phenomenon was observed in the presence of other different assayed drugs.

### Binding of [³⁵S]GTPγS in the absence (basal) and in the presence of different ligands of galanin

Using the microscope slides treated according to the process described in Example 1 as supports, a set of ligands of galanin receptors was studied for the purpose of determining the specific activity of each molecule (Figure 6). In said Figure, it can be observed how in some tissues (e.g., pituitary) the binding of [³⁵S]GTPγS in the presence of galanin, galnon, gal(2-11), M15, M35 and M40 increases, however the action of galnon is antagonized by M35 and M40 (Figure 7).

The obtained results clearly show that the microscope slides which have been treated with orthophosphoric acid according to the process described in Example 1 bind cell membranes such that the inserted proteins maintain their functionality. These membranes remain adhered to the support during the entire experiment. Therefore, the glass microscope slides treated according to the process described in Example 1 allow performing autoradiography experiments of [³⁵S]GTPγS (Figure 6 and 7). Furthermore, the arrays produced with the supports treated in this way have a lower nonspecific binding than the remaining surfaces treated with other compounds as can be observed in Figures 3, 4 and 5.

## Claims

1. A process for the chemical modification of a surface of a support, wherein said surface contains available hydroxyl groups and lacks metals or metallic alloys, comprising the treatment of said surface with orthophosphoric acid.

2. The process according to claim 1, wherein said surface is a glass surface or a surface of a polymeric material.

3. The process according to claim 1, comprising the treatment of said surface with an aqueous solution of orthophosphoric acid with a concentration comprised between approximately 10% by weight and approximately 85% by weight.

4. The process according to any of claims 1 to 3, wherein said treatment of the surface with orthophosphoric acid is carried out at a temperature comprised between approximately 20°C and approximately 100°C.

5. The process according to any of claims 1 to 4, further comprising one or more steps of washing and drying the surface to be treated prior to the treatment with orthophosphoric acid.

6. The process according to claim 5, wherein said step of washing is performed with an organic solvent.

7. The process according to claim 6, wherein said organic solvent is a short-chain alcohol or ketone.

8. A solid support comprising a surface treated according to the process defined in claims 1 to 7.

9. The solid support according to claim 8, configured in the form of a glass microscope slide.

10. A method for immobilizing a product in a surface of a solid support, wherein said product is selected from a biomolecule, a cell membrane, a liposome, a cell and a tissue section, and said surface contains available hydroxyl groups and lacks metals or metallic alloys, said method comprising the step of placing said product in contact with said glass surface treated according to the process defined in claims 1 to 7.

11. The method according to claim 10, further comprising subjecting the combination (immobilized product)-(treated surface) to a step of drying.

12. The method according to claim 10, wherein said biomolecule is a polynucleotide, a protein, a lipid, a combination of a lipid with a protein and/or with a polynucleotide, or a substrate or cofactor of said compounds.

13. A solid support comprising a surface treated according to the process defined in claims 1 to 7 and a product immobilized on said treated surface, wherein said product is selected from a biomolecule, a cell membrane, a liposome, a cell, a tissue section and mixtures thereof.

14. A solid support according to claim 13, configured in the form of a glass microscope slide.
